Europäisches Patentamt

(19) European Patent Office

Office européen des brevets

(11) Publication number: **0 090 368**
**B1**

(12) **EUROPEAN PATENT SPECIFICATION**

(45) Date of publication of patent specification: **02.09.87**

(21) Application number: **83102982.2**

(22) Date of filing: **25.03.83**

(51) Int. Cl.⁴: **A 61 K 33/04,** A 61 K 33/24,
A 61 K 45/06 // (A61K33/04,
33:24, 31:415)

(54) **Anthelmintic preparations.**

(30) Priority: **30.03.82 GB 8209347**

(43) Date of publication of application:
**05.10.83 Bulletin 83/40**

(45) Publication of the grant of the patent:
**02.09.87 Bulletin 87/36**

(84) Designated Contracting States:
**AT BE CH DE FR IT LI LU NL SE**

(56) References cited:
**EP-A-0 000 670
FR-A-2 263 780
GB-A-1 444 024**

**UNLISTED DRUGS, vol. 34, no. 8, August 1982,
abstract no. f, CHATHAM, NEW JERSEY (US).
"Panacur SC", page 128**

(73) Proprietor: **HOECHST UK LIMITED
Hoechst House Salisbury Road
Hounslow Middlesex TW4 6JH (GB)**

(72) Inventor: **Ganley, John Anthony
2 Thames Drive
Newport Pagnell Bucks (GB)**
Inventor: **McBeath, David George
2 The Woodlands
Cattle End Silverstone Northants (GB)**
Inventor: **McTaggart, Celia Margaret
9 Dunbar Close
Bletchley Milton Keyness Bucks (GB)**
Inventor: **Wells, Peter William
5 Calvert Close
Greens Norton Towcester Northants (GB)**

(74) Representative: **Meyer-Dulheuer, Karl-Hermann,
Dr. et al
HOECHST Aktiengesellschaft Zentrale
Patentabteilung Postfach 80 03 20
D-6230 Frankfurt/Main 80 (DE)**

## 0 090 368

**Description**

The present invention relates to anthelmintic preparations, particularly for the treatment of ruminants.

As clinical and sub-clinical nematode infections can cause serious economic loss, due to death, reduced weight gain and lowered milk yield, anthelmintic treatment of ruminants is now a routine practice. Treatment is also undertaken to reduce pasture larval contamination and thus aids in ensuring optimal grassland usage.

In some cases, however, even after anthelmintic treatment, animals still fail to thrive. It is now considered that this may be caused by mineral deficiencies resulting from soils and pastures deficient in minerals. Cobalt is one mineral that may be lacking in the diet of ruminants, particularly in the spring, when there is pasture dominance of rapidly growing grasses, which have a lower cobalt content than more slowly growing grasses. Cobalt participates in the production of vitamin $B_{12}$ in the rumen, and deficiency results in loss of apetite and loss of bodyweight, and the animal may finally waste away. In the ewe, in addition to weight loss, milk yield falls and there is a reduction in fleece quality. Selenium is also an essential element, but its exact metabolic function is uncertain. Deficiency is associated with muscular dystrophy, unthriftiness and infertility in female animals.

it has, therefore, been proposed to administer trace elements, particularly cobalt and selenium, to animals. Some of the trace elements, however, are toxic, and it is possible to overdose animals. This is particularly so with selenium, the oral $LD_{50}$ of which is only 0.5 mg/kg. The present invention is based on the surprising observation that a preparation comprising both selenium and an anthelmintic agent has a very greatly increased safety margin with regard to the selenium.

Accordingly, the present invention provides a veterinary preparation comprising an effective amount of an anthelmintic agent selected from fenbendazole, oxfendazole, albendazole, thiabendazole, parbendazole, cambendazole, oxibendazole, mebendazole and flubendazole, and a non-toxic amount of selenium salt selected from sodium selenate, potassium selenate, ammonium selenate, sodium selenite, potassium selenite, ammonium selenite, sodium selenide, potassium selenide and ammonium selenide, effective to prevent selenium deficiency in an animal. Preferably, the preparation also comprises a cobalt salt.

As a general rule, the less soluble a benzimidazole anthelmintic, the more active it is. Accordingly, fenbendazole, oxfendazole and albendazole are more active than thiabendazole, parbendazole and cambendazole and are, therefore, preferred.

Fenbendazole is a broad spectrum anthelmintic with excellent efficacy against adult, larval and egg stages of all important gastro-intestinal and respiratory nematodes of ruminants. In addition, it is effective against both scolices and segments of M: expansa, the cestode affecting sheep and cattle.

It is non-teratogenic, and so can be used safely in animals at any stage of pregnancy. Having no adverse effect on fertility, it can be used around the time of service and in the breeding male. It has a very high safety margin (sheep: 1000 times the recommended dose, cattle: 50 times the recommended dose). Moreover, it is non-toxic to handlers. Oxfendazole and albendazole have substantially the same spectrum of activity as fenbendazole, but have a lower therapeutic index. Fenbendazole (methyl 5-phenylthio-2-benzimidazolecarbamate) is particularly preferred as the anthelmintic agent for the preparation of the present invention.

A preparation of the invention may be in liquid or solid form. A liquid preparation may be a solution, but is generally a suspension, preferably a fairly mobile suspension. Other forms of preparation suitable for dosing animals may be used, for example, tablets, bolus tablets and pastes; and powders and granules for reconstitution as solutions or suspensions prior to use. Moreover, animals may be dosed by the addition of an anthelmintic agent, a selenium salt and, preferably also a cobalt salt, directly to the feed. A preparation of the invention, accordingly, may be in a form suitable for addition to an animal feedstuff. The invention further comprises an animal feedstuff that has been augmented by the incorporation of an anthelmintic agent, a selenium salt and, preferably, also a cobalt salt.

In a preparation of the invention the selenium is in the form of a selenate, selenite or selenide. Selenates are generally preferred as they are the most soluble, and sodium selenate is particularly preferred. The cobalt salt may be insoluble or soluble, examples of cobalt salts being cobalt carbonate, cobalt chloride, cobalt nitrate and cobalt sulphate. Cobaltous or cobaltic salts may be used. Soluble cobalt salts are preferred. In a liquid preparation, the selenium salt and, when present, the cobalt salt, is preferably in solution to ensure a uniform distribution of these elements throughout the preparation and therefore to improve the dose to dose reproducibility.

One or more further physiologically active substance(s) may be present in a preparation of the invention, for example, selected from vitamins and, especially, other trace elements, for example, copper, zinc and magnesium.

One or more carriers suitable for veterinary preparations may be present in addition to the active substances to aid manufacture and/or to confer desirable properties on the final preparation. Such materials include pH adjusters, pH buffers, wetting agents, suspending agents, thickening agents, stabilising agents, solubilisers, anti-microbial preservatives, lubricants and disintegration agents. Water is generally the basis for a solution or suspension.

In the veterinary preparation of this invention the anthelmintic agent is present—as regards sheep and

2

goats in an amount of from 100 to 400 parts by weight and the selenium salt is present in an amount, calculated as elemental selenium, of 1 to 20 parts by weight, whereby the preparation preferably additionally contains a non-toxic amount of cobalt salt effective to prevent cobalt deficiency in sheep and goats, wherein said cobalt salt is present in an amount, calculated as elemental cobalt, of 1 to 50 parts by weight.

When used for treating cattle, the anthelmintic agent is present in an amount of from 150 to 1500 parts by weight and the selenium salt is present in an amount, calculated as elemental selenium, of 0.75 to 18.75 parts by weight, whereby the preparation preferably additionally contains a non-toxic amount of cobalt salt effective to prevent cobalt deficiency in cattle, wherein said cobalt salt is present in an amount, calculated as elemental cobalt, of 0.75 to 37.5 parts by weight.

A suspension of the invention may be prepared by mixing the ingredients with water using a high speed mixer/homogeniser until a uniform dispersion is achieved. The pH of the suspension is usually adjusted to the range 5.5 to 6.5 and especially to 5.9 to 6.0 to optimise product stability. The suspension preferably includes ingredients to ensure an elegant product by retarding sedimentation and/or aiding resuspension on shaking, for example, a thixotropic agent, e.g. colloidal silicon dioxide, and/or a thickening agent, e.g. carboxymethyl cellulose. The selenium salt is preferably added as a solution to avoid dust problems on handling.

Other preparation forms, for example, tablets, powders and granules, may be prepared in a conventional manner.

In a suspension of the invention the fenbendazole content is, for example, in the range of from 10 to 40 mg/ml for sheep and goats, especially 25 mg/ml. For cattle, the fenbendazole content is, for example, from 20 to 200 mg/ml, especially 100 mg/ml. If a different anthelmintic agent is used, the dose should be substantially equivalent to that given above for fenbendazole. (The recommended doses of most anthelmintic agents for sheep, goats and cattle are generally well known).

The content of selenium in a suspension of the invention, calculated as elemental selenium is, for example, within the range of from 0.1 to 2.0 mg/ml, especially 0.4 mg/ml for sheep and goats, and within the range of for from 0.1 to 2.5 mg/ml, especially 2.0 mg/ml for cattle. The cobalt content, also calculated as the elemental substance is, for example, from 0.1 to 5.0 mg/ml, especially from 0.9 to 1.0 mg/ml for sheep and goats, and from 0.1 to 5.0 mg/ml, especially 2.5 mg/ml for cattle. If desired, however, the selenium content can be increased considerably, for example, up to 10 mg/ml, for sheep, goats and cattle.

A suspension of the invention for sheep and goats preferably comprises 25 mg of fenbendazole, 0.4 mg of elemental selenium and 0.95 mg of cobalt per ml. With this preparation, the recommended dose is 1 ml/5 kg body weight.

A suspension of the invention for cattle preferably comprises 100 mg of fenbendazole, 2.0 mg of elemental selenium and 2.5 mg of elemental cobalt per ml. The recommended dose of this preparation is 7.5 ml per 100 kg body weight.

In a tablet or bolus tablet of the invention, the doses of the various ingredients may be calculated pro rata from the doses given above, knowing the body weight of the intended recipient of the dose, for example, in a tablet suitable for administration to a 50 kg sheep, the fenbendazole content is generally from 100 to 400 mg, especially from 250 to 300 mg, the content of elemental selenium is generally from 1 to 20 mg, especially from 3 to 5 mg; and the cobalt content, calculated as elemental cobalt, is generally from 1 to 50 mg, especially from 9 to 10 mg. Tablets may be scored to assist the administration of divided doses to smaller animals, and multiple doses may be given to larger animals. The optimum dose of the various ingredients for solid preparations e.g. tablets and bolus tablets for cattle can be calculated similarly from the data given above for suspensions.

As indicated above, appropriate amounts of other anthelmintic agents can be readily calculated.

It will be appreciated that as an alternative to presenting the anthelmintic agent and the selenium, to an animal in a single preparation, these two components may be formulated separately and administered simultaneously or substantially simultaneously. The present invention accordingly also provides a veterinary pack which comprises an anthelmintic agent as defined above and, in conjunction or close juxtaposition therewith, a selenium salt as defined above. The pack preferably additionally contains a cobalt salt, said cobalt salt being separately packaged from said selenium salt and said anthelmintic agent and said anthelmintic agent, cobalt salt and selenium salt being combinable to form a veterinary preparation comprising an effective amount of an anthelmintic agent, a non-toxic amount of selenium salt effective to prevent selenium deficiency in an animal and a non-toxic amount of cobalt salt effective to prevent cobalt deficiency in an animal. The said anthelmintic agent is in the form of a veterinary preparation and is preferably in admixture or conjunction with a carrier suitable for use in a veterinary preparation. The preparation may be liquid or solid, and example of preparation forms are given above. The content of the said anthelmintic agent, the selenium salt and the cobalt salt for treating sheep and goats or cattle are preferably as indicated above. As indicated above, suspensions are generally preferred. The said selenium salt may be formulated either alone or in admixture or conjunction with a carrier suitable for veterinary use in solid or liquid form, for example, as tablet, bolus tablet or paste, as a solution or suspension, or as a powder or granules for reconstitution as a solution or suspension prior to use. It is advantageous to provide the said selenium in a unit dose form suitable for addition either to the suspension containing the said anthelmintic agent or to water, preferably purified water. Tablets and

# 0 090 368

sachets containing the appropriate amount of a selenium salt as defined above are examples of suitable dosage forms. The amounts of selenium to be incorporated can be calculated from the data given above.

The cobalt salt may be present either as a component of the anthelmintic preparation or as a component of the said selenium preparation.

Examples of both selenium salts and cobalt salts are given above.

The anthelmintic agent-containing preparation and the selenium-containing preparation should be provided in conjunction with one another or in close juxtaposition to one another for example, the two should be provided close to hand. Preferably, the two components are joined together, one is attached to the other, or both are provided in the same container. The selenium containing preparation, for example, tablets or a sachet containing a powder or granules for reconstitution, may be physically attached to the anthelmintic preparation, for example, a pack or bottle of tablets, or a bottle of liquid preparation. The two components may be provided together in a container, for example, a box or carton. Both components may be solid, both may be liquid, or one may be solid and one may be liquid. As indicated above, liquid forms may be preferred for the anthelmintic preparation, and a pack may comprise a bottle of anthelmintic suspension together with a bottle of selenium-containing preparation or a solid selenium preparation, for example, tablets or one or more sachets of powder or granules for reconstitution. Other preparation forms may be provided analogously.

The two preparations should be accompanied by instructions regarding their use. The instructions should indicate that the two preparations should be administered simultaneously or substantially simultaneously, with the selenium component preferably being administered immediately after the anthelmintic component.

The instructions should indicate that the selenium component can be added to the anthelmintic component, if this is appropriate. The selenium component can be administered separately. The appropriate dose of both components should be indicated. It may be convenient to provide the selenium component in units suitable for reconstitution in a predetermined amount of either water or anthelmintic suspension. The user may then decide whether to add the selenium component to the anthelmintic preparation, or to make it up with water and use it separately.

The recommended treatment times for sheep with a preparation of the invention are as follows:

Ewes:

in the period within 4—6 weeks of lambing. In addition, further treatments may be given approximately 4—6 weeks after lambing and prior to service—the former to assist in reduction of pasture larval contamination and the latter to improve bodily condition and, therefore, conception rate. Such additional treatments by providing cobalt and selenium will aid in body condition maintenance and improve fertility.

Lambs:

at 4—6 weeks of age and monthly thereafter to maintain optimal growth rate. Treatments may have to be more frequent in areas where Nematodirus infection occurs.

Bought-in sheep:

treat on arrival and thereafter as for ewes/lambs, but particularly (as with all sheep) when moving to clean pasture.

Treatment of cattle could be carried out as follows: Calves in their first grazing season: in a set stocked system, dose at 3 and 6 weeks post turnout and again at housing. Alternatively, dose and move to clean pasture in mid-summer, and dose again at housing.

Cattle in subsequent grazing seasons: dose prior to movement to clean pasture and at housing.

Cows: dose prior to calving.

As indicated above, we have found, surprisingly, that the safety margin for selenium administration is increased greatly when the selenium is combined with an anthelmintic agent, particularly fenbendazole. The $LD_{50}$ of elemental selenium per os in sheep is considered to be 0.5 mg/kg (W. M. Allen, Institute for Research on Animal Diseases, Compton), whereas we have found that administration of oral dose levels of elemental selenium of up to 2.0 mg/kg were tolerated, no signs of drug toxicity being observed in any of the treated animals.

This decrease in toxicity was quite unexpected, and we believe that there is a safety margin of at least 25 times the recommended dose of the preparation of the invention. This is of considerable practical importance, as there is risk of killing livestock by administering an overdose of selenium using trace element preparations currently available, whereas it is virtually impossible to administer a fatal dose of selenium using a preparation of the present invention. The following Examples illustrate the invention.

The anthelmintic agents denoted by their generic names are as follows.

Fenbendazole: 5-Phenylthio-2-benzimidazolecarbamic acid methyl ester

Oxfendazole: 5-Phenylsufinyl-2-benzimidazolecarbamic acid methyl ester

Albendazole: 5-Propylthio-2-benzimidazolecarbamic acid methyl ester

Oxidbendazole: 5-Propoxy-2-benzimidazolecarbamic acid methyl ester

Parbendazole: 5-Butyl-2-benzimidazolecarbamic acid methyl ester

4

Mebendazole: 5-Benzoyl-2-benzimidazolecarbamic acid methyl ester
Flubendazole: 5-(4-Fluorobenzoyl)-2-benzimidazolecarbamic acid methyl ester
Cambendazole: 2-(4-Thiazolyl)-5-benzimidazolecarbamic acid isopropyl ester
Thiabendazole: 2-(4-Thiazolyl)-benzimidazole

### Example 1
#### Composition per ml

| | |
|---|---|
| Fenbendazole | 25.000 mg |
| Colloidal silicon dioxide | 30.000 mg |
| Sodium methylhydroxybenzoate | 1.994 mg |
| Sodium propylhydroxybenzoate | 0.216 mg |
| Sodium carboxymethyl cellulose | 14.506 mg |
| Polyvinylpyrrolidone | 19.342 mg |
| Sodium citrate | 31.590 mg |
| Citric acid | 31.350 mg |
| Cobalt sulphate heptahydrate | 4.500 mg |
| Sodium selenate decahydrate 60% w/w solution | 3.167 mg |
| Purified water to | 1.0 ml |

Procedure

A mixing vessel was charged with water that had been filtered through a 0.45 µm filter. The following ingredients were added, with mixing to disperse them: citric acid, sodium citrate, cobalt sulphate, sodium selenate solution, sodium methylhydroxybenzoate, sodium propylhydroxybenzoate and colloidal silicon dioxide. The following ingredients were added via an in-line homogenisation unit: sodium carboxymethyl cellulose, polyvinylpyrrolidone and fenbendazole. Circulation through the in-line homogeniser was continued until a uniform dispersion was achieved. The pH was checked and adjusted to 5.9 with citric acid if necessary. The resulting pink suspension was filled into 2 litre containers.

This suspension contains 0.4 mg/ml elemental selenium and 0.94 mg/ml elemental cobalt.

Example 2
Determination of efficacy and toxicity levels
Materials and methods:

Animals: Twenty, six to eight month old lambs were made available for the purpose of this investigation. The lambs were allocated to five groups (n=4) on the basis of mean faecal egg counts determined on each of three successive days.

Prior to treatment, all lambs were weighed. Lambs were maintained at grass as a single group for the duration of the study.

Treatments:
Group 1:
Untreated controls
Group 2:
Treated with 5 mg/kg fenbendazole as Panacur 2.5% Suspension (Commercial batch, "Panacur", is a Trade Mark) Composition of Panacur 2.5%: as described in Example 1 with the omission of the selenium and cobalt components.
Group 3:
Treated with 5 mg/kg fenbendazole as a developmental formulation (see below)
Group 4:
Treated with 25 mg/kg fenbendazole as a developmental formulation (see below)
Group 5:
Treated with 50 mg/kg fenbendazole as a developmental formulation (see below)
All treatments were administered per os.

Developmental formulation
Procedure: As described in Example 1.
Composition per ml:

| | | |
|---|---|---|
| Fenbendazole | 25.000 | mg |
| Colloidal silicon dioxide | 30.000 | mg |
| Sodium methylhydroxybenzoate | 1.994 | mg |
| Sodium propylhydroxybenzoate | 0.216 | mg |
| Sodium carboxymethyl cellulose | 14.506 | mg |
| Polyvinylpyrrolidone | 19.542 | mg |
| Sodium citrate | 31.590 | mg |
| Citric acid | 31.350 | mg |
| Cobalt sulphate heptahydrate | 4.500 | mg |
| Sodium selenate decahydrate | 4.700 | mg |
| Purified water | to | 1.000 ml |

Groups 4 and 5 thus received five and ten times respectively the recommended dose of fenbendazole and cobalt, and 12.5 and 25 times respectively the recommended dose of selenium.

Parasitological examinations

Faecal egg counts were performed using the modified McMaster technique on samples taken directly from the rectum of lambs pre-treatment and at seven, eleven, eighteen and twenty five days post treatment.

Groups 1, 2 and 3 were slaughtered at eleven days post treatment and post-mortem examinations carried out. The abomasum, small intestine and large intestine of each animal were removed and the contents collected. The abomasal mucosa was digested in a pepsin/hydrochloric acid mixture for six hours. Numbers of helminth species present were calculated from aliquot samples of the organ contents (abomasal digests being examined in toto).

Clinical examinations

Clinical examinations were carried out on all lambs daily post treatment for the duration of the study.

Results

No signs of drug-associated toxicity were noted in any of the treated animals.

Faecal egg counts of all lambs are given in Table 1. Treatment with the commercially available or experimental formulations virtually eliminated faecal egg excretions.

Worm counts obtained from lambs in Groups 1—3 at slaughter are recorded in Table II.

A mixed nematode burden was present in the untreated lambs—*Haemonchus, Ostertagia* and *Trichostrongylus spp.* being present in the abomasum; *Cooperia, Nematodirus* and *Trichostrongylus spp.* in the small intestine and *Chabertia* and *Trichuris spp.* in the large intestine.

Treatment with both formulations virtually eliminated nematode burdens, there being no significant difference in efficacy between the two formulations.

Conclusions

1. Inclusion of the trace elements, cobalt and selenium, in Panacur 2.5% Suspension had no deleterious effect on anthelmintic efficacy.

2. Oral dose levels of up to 1.9 mg/kg of elemental cobalt (as $CoSo_4 \cdot 7H_2O$) and 2.0 mg/kg of elemental selenium (as $Na_2SeO_4 \cdot 10H_2O$) were tolerated, no signs of drug toxicity being noted in any of the treated lambs.

TABLE 1

| Group | Lamb No. | Weight (kg) | Faecal egg count (epg) on treatment day | | | | | | |
|---|---|---|---|---|---|---|---|---|---|
| | | | −3 | −2 | −1 | +7 | +11 | −18 | +25 |
| 1+ | 844 | 32.0 | 1500 | 2400 | 1950 | 2150 | 2350 | | |
| | 822 | 36.0 | 50 | 350 | 900 | 800 | 400 | | |
| | 866 | 26.5 | 650 | 1900 | 350 | 650 | 400 | | |
| | 812 | 32.0 | 50 | NIL | NIL | NIL | 50 | | |
| 2+ | 870 | 35.0 | 250 | 350 | 650 | NIL | NIL | | |
| | 823 | 33.0 | 700 | 350 | 1000 | NIL | NIL | | |
| | 856 | 32.0 | 1500 | 1650 | 1300 | NIL | NIL | | |
| | 880 | 38.0 | 1250 | 700 | 1100 | NIL | NIL | | |
| 3+ | 851 | 27.0 | 1050 | 1100 | 1650 | NIL | 50 | | |
| | 878 | 28.0 | 350 | 500 | 2000 | 50 | NIL | | |
| | 826 | 32.5 | 750 | 600 | 750 | NIL | NIL | | |
| | 2 | 28.5 | 650 | 650 | 1050 | NIL | NIL | | |
| 4 | 835 | 29.5 | 550 | 650 | 450 | NIL | NIL | NIL | NIL |
| | 843 | 31.0 | 700 | 800 | 1650 | NIL | NIL | NIL | NIL |
| | 824 | 34.5 | 700 | 1000 | 1050 | NIL | NIL | NIL | NIL |
| | 892 | 33.0 | NIL | NIL | NIL | NIL | NIL | NIL | NIL |
| 5* | 889 | 23.5 | NIL | NIL | NIL | NIL | NIL | NIL | NIL |
| | 896 | 33.5 | NIL | NIL | NIL | NIL | NIL | NIL | NIL |
| | 814 | 32.0 | NIL | NIL | NIL | NIL | NIL | NIL | NIL |
| | 811 | 25.0 | NIL | NIL | NIL | NIL | NIL | NIL | NIL |

Mean trichostronglyid egg counts in lambs pre and post treatment
+ Groups 1—3 slaughtered at day 11 post treatment.
* Group 5 lambs served for safety evaluation only.

TABLE II
Total worm counts at post mortems of groups 1—3

| Groups | Lamb No. | No. of worms recovered | | Small intestine | Large intestine |
|---|---|---|---|---|---|
| | | Abomasum | | | |
| | | Early larval stages | Adult/ Dev. stages | | |
| 1 | 844 | 82 | 19.400 | 11,900 | 320 |
| | 822 | 38 | 1,860 | 690 | 10 |
| | 866 | 25 | 16,700 | 5,300 | 80 |
| | 812 | 0 | 150 | 380 | 0 |
| | MEAN | 36 | 9,528 | 4,568 | 103 |
| 2 | 870 | 1 | 0 | 0 | 0 |
| | 823 | 0 | 0 | 0 | 0 |
| | 856 | 0 | 0 | 0 | 0 |
| | 880 | 0 | 0 | 0 | 0 |
| | MEAN | 0.3 | 0 | 0 | 0 |
| | % reduction compared to controls | 99.2 | 100 | 100 | 100 |
| 3 | 851 | 0 | 0 | 130 | 0 |
| | 878 | 0 | 0 | 0 | 0 |
| | 826 | 0 | 0 | 110 | 0 |
| | 2 | 0 | 0 | 0 | 0 |
| | MEAN | 0 | 0 | 60 | 0 |
| | % reduction compared to controls | 100 | 100 | 98.7 | 100 |

Example 3
Composition per millilitre

| | |
|---|---|
| Fenbendazole | 100.000 mg |
| Fumed silicon dioxide | 30.000 mg |
| Sodium methylhydroxybenzoate | 1.883 mg |
| Sodium propylhydroxybenzoate | 0.205 mg |
| Sodium carboxymethylcellulose | 13.720 mg |
| Polyvinylpyrrolidone (PVP) | 18.294 mg |
| Sodium citrate BP | 29.837 mg |
| Citric acid BP | 1.649 mg |
| Cobalt sulphate · $7H_2O$ | 11.925 mg |
| Sodium selenate · $10\ H_2O$ | 9.349 mg |
| Purified water | 880.000 mg |

Procedure

A 2,500 l mixing vessel was charged with water filtered through a 0.45 μm filter. The following

8

**0 090 368**

ingredients were added in order, with mixing to disperse them: citric acid, sodium citrate, cobalt sulphate, sodium selenate solution, sodium methylhydroxybenzoate, sodium propylhydroxybenzoate and colloidal silicon dioxide.

The following ingredients were added via the in-line homogenisation unit: sodium carboxymethylcellulose, polyvinylpyrrolidone and fenbendazole. Circulation through the in-line homogeniser was continued until a uniform dispersion was achieved. The pH was checked and adjusted to 5.9 with citric acid if necessary. The resulting pink suspension was filled into 2 litre containers.

Each millilitre of this suspension contains 2.0 mg elemental selenium and 2.5 mg elemental cobalt. This suspension is suitable for cattle. The suggested dose is 7.5 ml per 100 kg body weight.

### Example 4
#### Composition per ml

| | |
|---|---|
| Albendazole | 25.000 mg |
| Colloidal silicon dioxide | 30.000 mg |
| Sodium methylhydroxybenzoate | 1.994 mg |
| Sodium propylhydroxybenzoate | 0.216 mg |
| Sodium carboxymethyl cellulose | 14.506 mg |
| Polyvinylpyrrolidone | 19.342 mg |
| Sodium citrate | 31.590 mg |
| Citric acid | 31.350 mg |
| Cobalt sulphate $7H_2O$ | 4.500 mg |
| Sodium selenate $\cdot 10H_2O$ | 3.167 mg |
| Purified water | to 1.0 ml |

Procedure

The preparation was made up by a procedure analogous to that described in Example 3.

Each ml of suspension contains 0.4 mg of elemental selenium and 0.95 mg of elemental cobalt. This suspension is suitable for administration to sheep or goats. The suggested dose is 1 ml per 5 kg body weight.

### Example 5
#### Composition per ml

| | |
|---|---|
| Albendazole | 100.000 mg |
| Fumed silicon dioxide | 30.000 mg |
| Sodium methylhydroxybenzoate | 1.883 mg |
| Sodium propylhydroxybenzoate | 0.205 mg |
| Sodium carboxymethyl cellulose | 13.720 mg |
| Polyvinylpyrrolidone (PVP) | 18.294 mg |
| Sodium citrate BP | 29.837 mg |
| Citric acid BP | 1.649 mg |
| Cobalt sulphate $\cdot 7H_2O$ | 11.925 mg |
| Sodium selenate $\cdot 10H_2O$ | 9.349 mg |
| Purified water | 880.000 mg |

Procedure

The composition was made up using a procedure analogous to that described in Example 3.

Each millilitre of suspension contains 2.0 mg of elemental selenium and 2.5 mg of elemental cobalt.

9

**0 090 368**

This suspension is suitable for administration to cattle. The suggested dose is 7.5 mg per 100 kg body weight.

Example 6
Composition per ml

| | |
|---|---|
| Oxfendazole | 22.650 mg |
| Colloidal silicon dioxide | 30.000 mg |
| Sodium methylhydroxybenzoate | 1.994 mg |
| Sodium propylhydroxybenzoate | 0.216 mg |
| Sodium carboxymethyl cellulose | 14.506 mg |
| Polyvinylpyrrolidone | 19.342 mg |
| Sodium citrate | 31.590 mg |
| Citric acid | 31.350 mg |
| Cobalt sulphate · 7H$_2$O | 4.500 mg |
| Sodium selenate · 10H$_2$O | 3.167 mg |
| Purified water | to 1.0 ml |

Procedure

The above composition was made up using a procedure analogous to that described in Example 3. Each millilitre of this suspension contains 0.4 mg of elemental selenium and 0.94 mg of elemental cobalt, and is suitable for administration to sheep or goats. The suggested dose is 1 ml per 5 kg body weight.

Example 7
Composition per millilitre

| | |
|---|---|
| Oxfendazole | 90.600 mg |
| Fumed silicon dioxide | 30.000 mg |
| Sodium methylhydroxybenzoate | 1.883 mg |
| Sodium propylhydroxybenzoate | 0.205 mg |
| Sodium carboxymethyl cellulose | 13.720 mg |
| Polyvinylpyrrolidone (PVP) | 18.294 mg |
| Sodium citrate BP | 29.837 mg |
| Citric acid BP | 1.649 mg |
| Cobalt sulphate · 7H$_2$O | 17.887 mg |
| Sodium selenate · 10 H$_2$O | 14.023 mg |
| Purified water | 880.000 mg |

Procedure

The above composition was made up using a procedure analogous to that described in Example 3. Each millilitre of this suspension contains 3.0 mg of elemental selenium and 3.75 mg of elemental cobalt. The suspension is suitable for administration to cattle, and the recommended dose is 5 ml per 100 kg body weight.

10

Example 8
Composition per millilitre

| | |
|---|---|
| Fenbendazole | 100.000 mg |
| Fumed silicon dioxide | 30.000 mg |
| Sodium methylhydroxybenzoate | 1.883 mg |
| Sodium propylhydroxybenzoate | 0.205 mg |
| Sodium carboxymethylcellulose | 13.720 mg |
| Polyvinylpyrrollidone | 18.294 mg |
| Sodium citrate | 29.837 mg |
| Citric acid | 1.649 mg |
| Cobalt sulphate · 7H$_2$O | 11.925 mg |
| Sodium selenite · 5 H$_2$O | 6.662 mg |
| Purified water | to 100.000 mg |

Procedure

The preparation was made up by a procedure analogous to that described in Example 3.

Each millilitre of suspension contains 2.0 mg of elemental selenium and 2.5 mg of elemental cobalt. The suspension is suitable for administration to cattle at a recommended dose of 7.5 mg per 100 kg body weight.

Example 9

(A) The preparation of Example 1 was made up, omitting the selenium and cobalt components, which were replaced by the equivalent weight of purified water. The resulting suspension was filled into 2 litre bottles. Sachets were prepared, each containing 4.5 g of cobalt sulphate · 7H$_2$O and 3.17 g of sodium selenate · 10H$_2$O. The bottles and sachets were packaged into boxes at the rate of one bottle and two sachets per box, together with instructions

(a) to mix the contents of one sachet with one litre of suspension before administration to sheep at a recommended dose of 1 ml per 5 kg body weight, or

(b) to mix the contents of each sachet with 1 litre of water, preferably purified water, and to administer the resulting preparation to sheep substantially simultaneously with the anthelmintic suspension. Each being administered at a recommended dose of 1 ml per 5 kg body weight. The selenium/cobalt preparation is preferably administered after the anthelmintic preparation. The contents of the sachet may be made up in a different volume of water, if this is more convenient. The recommended dose should then be calculated pro rata.

(B) The preparation of Example 6 was made up analogously to the procedure described in (a) above, i.e. omitting the selenium and cobalt components, which were packaged into sachets as described in (A). The anthelmintic suspension was filled into 5 litre bottles, and bottles and sachets were packaged into boxes at the rate of one bottle and 5 sachets per box. Instructions as set out in (A) were included in each box.

(C) The preparation of Example 4 was made up as described in (A) above, but only the selenium component of Example 4 was replaced by purified water. Sachets were made up as described in (A) above, containing 3.17 g of sodium selenate · 10 H$_2$O. Bottles and sachets were packaged in boxes at the rate of one 2 litre bottle of suspension and two sachets per box. Instructions were included in each box, as specified in (A) above.

(D) The preparation of Example 1 was made up as described in (A) above, but only the selenium component of Example 1 was replaced by purified water. Sachets were made up as described in (A), containing 3.17 g of sodium selenate · 10H$_2$O. Bottles and sachets were packaged into boxes at the rate of one 2 litre bottle of suspension and two sachets per box. Instructions were included in each box, as specified in (A) above.

Example 10

(A) The preparation of Example 3 was made up with the omission of the selenium and cobalt components, which were replaced by the equivalent weight of purified water. The resulting suspension was filled into 5 litre bottles. Sachets were prepared, each containing 11.9 g of cobalt sulphate 7 H$_2$O and 9.3 g of sodium selenate · 10 H$_2$O. The bottles and sachets were packaged into boxes at the rate of one bottle and five sachets per box, together with instructions to mix the contents of each sachet with either (A) one litre of the anthelmintic suspension or (b) one litre of water, preferably purified water. In the case of (a), the resulting combined preparation is to be administered to cattle at a recommended dose of 7.5 ml per 100 kg body weight. (It is suggested that for convenience, the contents of all five sachets can be admixed with the contents of the 5 litre bottle of suspension). In the case of (b), the instructions point out the requirement for the substantially simultaneous administration of the anthelmintic suspension and the selenium/cobalt preparation, and suggest that the selenium/cobalt preparation is administered immediately after the anthelmintic suspension. The recommended dose for each preparation is 7.5 ml per 100 kg body weight. The instructions also point out that in case (b) the sachets may be made up in a different volume of water, if desired. The recommended dose should then be calculated pro rata.

(B) The preparation described in Example 5 was made up as described in (A) above i.e. with the replacement of the selenium and cobalt components by the equivalent weight of purified water. Sachets containing the selenium and cobalt salts were made up as described in (A) above. The anthelmintic suspension, filled into 2 litre bottles, and the sachets, were packaged into boxes at the rate of one bottle and two sachets per box, together with instructions as set out in (A) above.

(C) The preparation of Example 7 was made up as described in (A) above but with the replacement of the selenium component only by the equivalent weight of purified water, and filled into 5 litre bottles. Sachets were prepared, each containing 14.02 g of sodium selenate · $10H_2O$. The bottles and sachets were packaged into boxes at the rate of one bottle and five sachets per box, together with instructions. The instructions were as in (A) above except that the recommended dose of the combined preparation or of one selenium sachet made up in one litre of water is 5 ml per 100 kg body weight.

(D) The preparation of Example 8 was made up as described in (A) above, i.e. with the replacement of the selenium and cobalt components with the equivalent weight of purified water, and filled into 5 litre bottles. Sachets were prepared, each containing 6.662 g of sodium selenite. 5 $H_2O$ and 11.925 g of cobalt sulphate · 7 $H_2O$. The bottles and sachets were packaged into boxes at the rate of one bottle and five sachets per box, together with instructions as specified in (A) above.

(E) The preparation of Example 3 was made up as described in (A) above but with the replacement of only the selenium component and not the con cobalt component by the equivalent weight of purified water, and filled into 5 litre bottles. Sachets were prepared, each containing 9.35 g of sodium selenate · $10H_2O$. Sachets and bottles were packaged into boxes at the rate of one bottle and five sachets per box. Instructions as set out in (A) above were also included in each box.

Example 11

The preparation of Example 3 was tested for anthelmintic efficacy in cattle. There was also used a comparative preparation that did not contain selenium or cobalt.

|  Comparison preparation: Composition per ml. | |
| --- | --- |
| Fenbendazole | 100.000 mg |
| Fumed silicon dioxide | 30.000 mg |
| Sodium methylhydroxybenzoate | 1.883 mg |
| Sodium propylhydroxybenzoate | 0.205 mg |
| Sodium carboxymethylcellulose | 13.720 mg |
| Polyvinylpyrrolidone (PVP) | 18.294 mg |
| Sodium citrate BP | 29.837 mg |
| Citric acid BP | 1.649 mg |
| Purified water | 901.274 mg |

The comparison preparation was made up as described in Example 3.

Method

A group of 23 heifers was made available for this study. The cattle had been at grass over the summer and had recently been housed when sampled for allocation to treatment group. Cattle in all three groups were housed in one pen.

Faeces samples were collected from all the heifers on two successive days, and were examined for the presence of worm eggs. On the basis of these worm egg counts the heifers were allocated to one of three treatment groups as shown in Table 1.

12

On the day after second sampling, the cattle were weighed and treated appropriately as indicated in Table 1. Thereafter, faeces samples were collected from the cattle on days 4, 7 and 17 following treatment. Faecal wogm egg counts were carried out using the modified McMaster technique.

Results

The nematode eggs observed in the faeces samples were of the strongyle species and the results of faecal egg counts for all the heifers are shown in Table 2. Both anthelmintic preparations were shown to result in elimination of faecal egg excretion.

Conclusions

The inclusion of selenium and cobalt does not affect the anthelmintic efficacy of the preparation of. Example 3.

TABLE III

Faecal worm egg counts prior to treatment

| Animal number | | Day-2 | Day-1 | Weight (kg) | Dose (ml) | Treatment |
|---|---|---|---|---|---|---|
| L1282 | | — | 50 | 350 | 27 | |
| B1175 | | 200 | 250 | 380 | 28 | |
| Y1252 | | 400 | 750 | 400 | 30 | |
| R1396 | | — | — | 420 | 32 | Preparation |
| Q1342 | | 450 | 550 | 415 | 31 | of Example 3 |
| W1331 | | 500 | 350 | 330 | 26 | |
| L1279 | | 300 | 250 | 330 | 26 | |
| W1307 | | 550 | 400 | 380 | 28 | |
| | X | 300 | 325 | | | |
| Y1267 | | — | — | 370 | 27 | |
| L1246 | | 550 | 450 | 400 | 30 | |
| W1372 | | — | — | 340 | 26 | Comparative |
| L1245 | | 250 | 150 | 400 | 30 | preparation |
| L1271 | | 450 | 500 | 370 | 37 | |
| W1342 | | 450 | 300 | 360 | 27 | |
| | X | 283 | 233 | | | |
| L1184 | | 50 | 100 | 440 | — | |
| L1237 | | 650 | 400 | 440 | — | |
| W1340 | | 250 | 200 | 300 | — | Controls |
| W1336 | | 450 | 600 | 360 | — | untreated |
| W1281 | | 100 | — | 380 | — | |
| L1232 | | — | 50 | 370 | — | |
| W1339 | | 300 | 200 | 330 | — | |
| | X | 257 | 221 | | | |

**0 090 368**

TABLE IV

Faecal worm egg counts
following treatments

| Animal number | Treatment | Day 4 | Day 7 | Day 19 |
|---|---|---|---|---|
| L1282 | | — | — | — |
| B1175 | | — | — | — |
| Y1252 | | — | — | — |
| R1395 | Preparation of | — | — | — |
| Q1342 | Example 3 | — | — | — |
| W1331 | | — | — | — |
| L1279 | | — | — | — |
| W1307 | | — | — | — |
| | | | | |
| Y1267 | | — | — | — |
| L1246 | | — | — | — |
| W1372 | Comparative | — | — | — |
| L1245 | preparation | — | — | — |
| L1271 | | — | — | — |
| W1342 | | — | — | — |
| | | | | |
| L1184 | | 100 | 50 | * |
| L1237 | | 700 | 550 | 550 |
| W1340 | | 200 | 200 | 300 |
| W1336 | Controls | 450 | 450 | 400 |
| W1281 | untreated | 50 | — | — |
| L1232 | | — | — | * |
| W1339 | | 300 | 350 | 200 |
| X | | 257 | 228 | 290 |

\* Not sampled

## Example 12

The safety of the composition of Example 3 on oral administration to cattle was investigated: A total of 16 cattle were treated orally with the preparation of Example 3. Eight Friesian heifers weighing between 330 and 420 kg were treated with the preparation at the recommended dose of 7.5 ml per 100 kg body weight. Four Friesian cattle weighing between 930 and 990 kg were treated orally with the preparation at five times the recommended dose i.e. 37.5 ml ·per 100 kg. A further four Friesian cattle weighing between 870 and 1030 kg were treated orally with the preparation at ten times the recommended dose i.e. 75 mg per 100 kg. Table 1 shows the computed and actual dose administered relative to live weight.

The cattle were observed daily for a period of 21 days following treatment for any signs at adverse effect.

Results and conclusions

No signs of adverse toxicity were observed in cattle treated with up to ten times the recommended dose of the formulation.

Oral administration of up to 20 mg/kg of selenium and 25 mg/kg of cobalt in combination with 75 mg/kg of fenbendazole was carried out in cattle with no adverse effect observed.

14

TABLE V

Volumes of preparation of Example 3 administered in safety tests.

| Animal number | Weight (kg) | Calculated dose (ml) | Dose administered (ml) |
|---|---|---|---|
| L1282 | 350 | 26.2 | 27 |
| B11 | 380 | 28.5 | 28 |
| Y1252 | 400 | 30.0 | 30 |
| R1396 | 420 | 31.5 | 32 |
| Q1342 | 415 | 31.1 | 31 |
| W1331 | 330 | 24.8 | 26 |
| L1279 | 330 | 24.8 | 26 |
| W1307 | 380 | 28.5 | 28 |
| 2 | 990 | 372 | 380 |
| 7 | 950 | 357 | 360 |
| 71 | 930 | 349 | 360 |
| 10 | 930 | 349 | 360 |
| 65 | 870 | 652 | 660 |
| 17 | 1030 | 774 | 780 |
| 87 | 960 | 721 | 720 |
| 90 | 925 | 695 | 700 |

**Claims**

1. A veterinary preparation comprising an effective amount of an anthelmintic agent selected from fenbendazole, oxfendazole, albendazole, thiabendazole, parbendazole, cambendazole, oxibendazole, mebendazole and flubendazole, and a non-toxic amount of selenium salt selected from sodium selenate, potassium selenate, ammonium selenate, sodium selenite, potassium selenite, ammonium selenite, sodium selenide, potassium selenide and ammonium selenide, effective to prevent selenium deficiency in an animal.

2. The veterinary preparation of Claim 1, wherein said anthelmintic agent and selenium salt are in admixture.

3. The veterinary preparation of Claim 1 or 2, additionally comprising a solid or liquid carrier suitable for use in a veterinary preparation.

4. The veterinary preparation of Claim 1 or 2 or 3, additionally comprising a non-toxic amount of cobalt salt effective to prevent cobalt deficiency in an animal, said cobalt salt preferably being a soluble cobalt salt selected from cobalt chloride, cobalt carbonate, cobalt nitrate and cobalt sulphate.

5. The veterinary preparation of 4, wherein said anthelmintic agent is fenbendazole.

6. A veterinary preparation for sheep and goats comprising an effective amount of an anthelmintic agent selected from fenbendazole, oxfendazole, albendazole, thiabendazole, parbendazole, cambendazole, oxibendazole, mebendazole and flubendazole, and a non-toxic amount of selenium salt selected from sodium selenate, potassium selenate, ammonium selenate, sodium selenite, potassium selenite, ammonium selenite, sodium selenide, potassium selenide and ammonium selenide, effective to prevent selenium deficiency in sheep and goats, wherein said anthelmintic agent is present in an amount of from 100 to 400 parts by weight; and wherein said selenium salt is present in an amount, calculated as elemental selenium, of 1 to 20 parts by weight, the preparation preferably additionally comprising a non-toxic amount of cobalt salt effective to prevent cobalt deficiency in sheep and goats, wherein said cobalt salt is present in an amount, calculated as elemental cobalt, of 1 to 50 parts by weight.

7. A veterinary preparation for cattle comprising an effective amount of an anthelmintic agent selected from fenbendazole, oxfendazole, albendazole, thiabendazole, parbendazole, cambendazole, oxibendazole, mebendazole and flubendazole, and a non-toxic amount of selenium salt selected from sodium selenate, potassium selenate, ammonium selenate, sodium selenite, potassium selenite, ammonium selenite, sodium selenide, potassium selenide and ammonium selenide, effective to prevent selenium deficiency in cattle, wherein said anthelmintic agent is present in an amount of from 150 to 1500 parts by weight; and wherein said selenium salt is present in an amount, calculated as elemental selenium, of 0.75 to 18.75 parts by weight, the preparation preferably additionally comprising a non-toxic amount of cobalt salt effective to prevent cobalt deficiency in cattle, wherein said cobalt salt is present in an amount, calculated as elemental cobalt, of 0.75 to 37.5 parts by weight.

8. A veterinary pack comprising an anthelmintic agent according to Claim 1 and a selenium salt according to Claim 1, said anthelmintic agent and said selenium salt being separately packaged and said

15

anthelmintic agent and said selenium salt being combinable to form a veterinary preparation comprising an effective amount of the anthelmintic agent and a non-toxic amount of the selenium salt effective to prevent selenium deficiency in an animal, the pack preferably additionally comprising a cobalt salt, said cobalt salt being separately packaged from said selenium salt and said anthelmintic agent and said anthelmintic agent, cobalt salt and selenium salt being combinable to form a veterinary preparation comprising an effective amount of an anthelmintic agent, a non-toxic amount of selenium salt effective to prevent selenium deficiency in an animal and a non-toxic amount of cobalt salt effective to prevent cobalt deficiency in an animal.

9. A veterinary pack for sheep and goats comprising an anthelmintic agent according to Claim 1 and a selenium salt according to Claim 1, said anthelmintic agent and said selenium salt being separately packaged and said anthelmintic agent and said selenium salt being combinable to form a veterinary preparation for sheep and goats comprising an effective amount of the anthelmintic agent and a non-toxic amount of the selenium salt effective to prevent selenium deficiency in an animal; wherein said anthelmintic agent is present in said pack in an amount of from 100 to 400 parts by weight; and wherein said selenium salt is present in said pack in an amount, calculated as elemental selenium, of 1 to 20 parts by weight, the pack preferably additionally comprising a cobalt salt, said cobalt salt being separately packaged from said selenium salt and said anthelmintic agent and said anthelmintic agent, cobalt salt and selenium salt being combinable to form a veterinary preparation for sheep and goats comprising an effective amount of an anthelmintic agent, a non-toxic amount of selenium salt effective to prevent selenium deficiency in an animal and a non-toxic amount of cobalt salt effective to prevent cobalt deficiency in an animal; wherein said cobalt salt is present in said pack in an amount, calculated as elemental cobalt, of 1 to 50 parts by weight.

10. A veterinary pack for cattle comprising an anthelmintic agent according to Claim 1 and a selenium salt according to Claim 1, said anthelmintic agent and said selenium salt being separately packaged and said anthelmintic agent and said selenium salt being combinable to form a veterinary preparation for cattle comprising an effective amount of the anthelmintic agent and a non-toxic amount of the selenium salt effective to prevent selenium deficiency in an animal; wherein said anthelmintic agent is present in said pack in an amount of from 150 to 1500 parts by weight; and wherein said selenium salt is present in said pack in an amount, calculated as elemental selenium, of 0.75 to 18.75 parts by weight, the pack preferably additionally comprising a cobalt salt, said cobalt salt being separately packaged from said selenium salt and said anthelmintic agent and said anthelmintic agent, cobalt salt and selenium salt being combinable to form a veterinary preparation for cattle comprising an effective amount of an anthelmintic agent, a non-toxic amount of selenium salt effective to prevent selenium deficiency in an animal and a non-toxic amount of cobalt salt effective to prevent cobalt deficiency in an animal; wherein said cobalt salt is present in said pack in an amount, calculated as elemental cobalt, of 0.75 to 37.5 by weight.

**Patentansprüche**

1. Veterinärmedizinische Zubereitung enthaltend ein unter Fenbendazol, Oxfendazol, Albendazol, Thiabendazol, Parbendazol, Cambendazol, Oxibendazol, Mebendazol und Flubendazol ausgewähltes Anthelminthikum in wirksamer Menge sowie ein unter Natriumselenat, Kaliumselenat, Ammoniumselenat, Natriumselenit, Kaliumselenit, Ammoniumselenit, Natriumselenid, Kaliumselenid und Ammoniumselenid ausgewähltes Selensalz in nicht-toxischer, zur Verhinderung eines Selenmangels in einem Tier wirksamer Menge.

2. Veterinärmedizinische Zubereitung nach Anspruch 1, worin jenes Anthelminthikum und Selensalz in Abmischung vorliegen.

3. Veterinärmedizinische Zubereitung nach Anspruch 1 oder 2, zusätzlich enthaltend einen zur Verwendung in einer veterinärmedizinischen Zubereitung geeigneten festen oder flüssigen Träger.

4. Veterinärmedizinische Zubereitung nach Anspruch 1, 2 oder 3, zusätzlich enthaltend ein Kobaltsalz in nichttoxischer, zur Verhinderung eines Kobaltmangels in einem Tier wirksamer Menge, wobei jenes Kobaltsalz vorzugsweise ein unter Kobaltchlorid, Kobaltcarbonat, Kobaltnitrat und Kobaltsulfat ausgewähltes lösliches Kobaltsalz ist.

5. Veterinärmedizinische Zubereitung nach Anspruch 4, worin als solches Anthelminthikum Fenbendazol vorliegt.

6. Veterinärmedizinische Zubereitung für Schafe und Ziegen, enthaltend ein unter Fenbendazol, Oxfendazol, Albendazol, Thiabendazol, Parbendazol, Cambendazol, Oxibendazol, Mebendazol und Flubendazol ausgewähltes Anthelminthikum in wirksamer Menge sowie ein unter Natriumselenat, Kaliumselenat, Ammoniumselenat, Natriumselenit, Kaliumselenit, Ammoniumselenit, Natriumselenid, Kaliumselenid und Ammoniumselenid ausgewähltes Selensalz in nicht-toxischer, zur Verhinderung eines Selenmangels in Schafen und Ziegen wirksamer Menge, worin jenes Anthelminthikum in einer Menge von 100 bis 400 Gewichtsteilen und jenes Selensalz in einer Menge von 1 bis 20 Gewichtsteilen, berechnet als elementares Selen, vorliegt, wobei die Zubereitung vorzugsweise zusätzlich ein Kobaltsalz in nicht-toxischer, zur Verhinderung eines Kobaltmangels in Schafen und Ziegen wirksamen Menge enthält, worin jenes Kobaltsalz in einer Menge von 1 bis 50 Gewichtsteilen, berechnet als elementares Kobalt, vorliegt.

7. Veterinärmedizinische Zubereitung für Rinder, enthaltend ein unter Fenbendazol, Oxfendazol, Albendazol, Thiabendazol, Parbendazol, Cambendazol, Oxibendazol, Mebendazol und Flubendazolausgewähltes Anthelminthikum in wirksamer Menge sowie ein unter Natriumselenat, Kaliumselenat, Ammoniumselenat, Natriumselenit, Kaliumselenit, Ammoniumselenit, Natriumselenid, Kaliumselenid und Ammoniumselenid ausgewähltes Selensalz in nicht-toxischer, zur Verhinderung eines Selenmangels in Rindern wirksamen Menge, worin jenes Anthelminthikum in einer Menge von 150 bis 1 500 Gewichtsteilen und jenes Selensalz in einer Menge von 0,75 bis 18,75 Gewichtsteilen, berechnet als elementares Selen, vorliegt, wobei die Zubereitung vorzugsweise zusätzlich ein Kobaltsalz in nicht-toxischer, zur Verhinderung eines Kobaltsmangels in Rindern wirksamen Menge enthält, worin jenes Kobaltsalz in einer Menge von 0,75 bis 37,5 Gewichtsteilen, berechnet als elementares Kobalt, vorliegt.

8. Veterinärmedizinische Packung enthaltend ein Anthelminthikum nach Anspruch 1 und ein Selensalz nach Anspruch 1, wobei jenes Anthelminthikum und jenes Selensalz getrennt verpackt sind und jenes Anthelminthikum und jenes Selensalz zur Bildung einer eine wirksame Menge des Anthelminthikums und eine nicht-toxische, zur Verhinderung eines Selenmangels in einem Tier wirksamen Menge des Selensalzes enthaltende veterinärmedizinischen Zubereitung kombiniert werden könne, wobei die Packung vorzugsweise zusätzlich ein Kobaltsalz enthält, jenes Kobaltsalz getrennt von jenem Selensalz verpackt wird und jenes Anthelminthikum, Kobaltsalz und Selensalz zur Bildung einer eine wirksame Menge des Anthelminthikums, eine nicht-toxische, zur Verhinderung eines Selenmangels in einem Tier wirksame Menge des Selensalzes und eine nicht-toxische, zur Verhinderung eines Kobaltmangels in einem Tier wirksame Menge des Kobaltsalzes enthaltenden veterinärmedizinischen Zubereitung kombiniert werden können.

9. Veterinärmedizinische Packung für Schafe und Ziegen, enthaltend ein Anthelminthikum nach Anspruch 1 und ein Selensalz nach Anspruch 1, wobei jenes Anthelminthikum und jenes Selensalz getrennt voneinander verpackt sind und jenes Anthelminthikum und jenes Selensalz zur Bildung einer eine wirksame Menge des Anthelminthikums und eine nicht-toxische zur Verhinderung eines Selenmangels in einem Tier wirksame Menge des Selensalzes enthaltenden veterinärmedizinischen Zubereitung für Schafe und Ziegen kombiniert werden können worin jenes Anthelminthikum in jener Packung in einer Menge von 100 bis 400 Gewichtsteilen vorliegt und, worin jenes Selensalz in jener Packung in einer Menge von 1 bis 20 Gewichtsteilen, berechnet als elementares Selen, vorliegt, wobei die Packung vorzugsweise zusätzlich ein Kobaltsalz enthält, jenes Kobaltsalz getrennt von jenem Selensalz und jenem Anthelminthikum verpackt ist und jenes Anthelminthikum, Kobaltsalz und Selensalz zur Bildung einer eine wirksame Menge eines Anthelminthikums, eine nicht-toxische, zur Verhinderung eines Selenmangels in einem Tier wirksamen Menge des Selensalzes und eine nicht-toxische zur Verhinderung eines Kobaltmangels in einem Tier wirksame Menge des Kobaltsalzes enthaltende veterinärmedizinische Zubereitung für Schafe und Ziegen kombiniert werden könne, worin jenes Kobaltsalz in jener Packung in einer Menge von 1 bis 50 Gewichtsteilen, bereichnet als elementares Kobalt, vorliegt.

10. Veterinärmedizinische Packung für Rinder, enthaltend ein Anthelminthikum nach Anspruch 1 und ein Selensalz nach Anspruch 1, wobei jenes Anthelminthikum und jenes Selenslaz getrennt verpackt sind und jenes Anthelminthikum und jenes Selensalz zur Bildung einer eine wirksame Menge des Anthelminthikums und eine nicht-toxische, zur Verhinderung eines Selenmangels in einem Tier wirksame Menge des Selensalzes enthaltende veterinärmedizinische Zubereitung für Rinder kombiniert werden können, worin jenes Anthelminthikum in jener Packung in einer Menge von 150 bis 1 500 Gewichts-teilen und jenes Selenzals in jener Packung in einer Menge von 0,75 bis 18,75 Gewichtsteilen, berechnet als elementares Selen, vorliegt, wobei die Packung vorzugsweise zusätzlich ein Kobaltsalz enthält, jenes Kobaltsalz getrennt von jenem Selensalz und jenem Anthelminthikum verpackt ist und jenes Anthelminthikum, Kobaltsalz und Selensalz zur Bildung einer eine wirksame Menge eines Anthelminthikums, einer nichttoxischen, zur Verhinderung eines Selenmangels in einem Tier wirksame Menge des Selensalzes und eine nicht-toxische, zur Verhinderung eines Kobaltmangels in einem Tier wirksame Menge des Kobaltsalzes enthaltenden veterinärmedizinischen Zubereitung kombiniert werden können, worin jenes Kobaltsalz in jener Packung in einer Menge von 0,75 bis 37,5 Gewichtsteilen, berechnet als elementares Kobalt, vorliegt.

**Revendications**

1. Préparation vétérinaire comprenant une quantité efficace d'un agent anthelmintique choisi parmi le fenbendazole, l'oxfendazole, l'albendazole, le thiabendazole, le parbendazole, le cambendazole, l'oxibendazole, le mébendazole et le flubendazole, et une quantité non-toxique d'un sel de sélénium choisi parmi le sélénate de sodium le sélénate de potassium le sélénate d'ammonium, le sélénite de sodium, le sélénite de potassium, le sélénite d'ammonium, le séléniure de sodium, le séléniure de potassium et le séléniure d'ammonium, efficace pour prévenir une carence en sélénium chez un animal.

2. Préparation vétérinaire suivant la revendication 1, dans laquelle ledit agent anthelmintique et le sel de sélénium sont mélangés.

3. Préparation vétérinaire suivant la revendication 1 ou 2, comprenant en outre un véhicule solide ou liquide approprié pour être utilisé dans une préparation vétérinaire.

4. Préparation vétérinaire suivant la revendication 1, 2 ou 3, comprenant en outre une quantité non toxique d'un sel de cobalt efficace pour prévenir une carence en cobalt chez un animal, ledit sel de cobalt

**0 090 368**

étant de préférence un sel soluble choisi parmi le chlorure de cobalt, le carbonate de cobalt, le nitrate de cobalt et le sulfate de cobalt.

5. Préparation vétérinaire suivant la revendication 4, dans lequelle ledit agent anthelmintique est du fenbendazole.

6. Préparation vétérinaire pour les moutons et les chèvres, comprenant une quantité efficace d'un agent anthelmintique choisi parmi le fenbendazole, l'oxfendazole, l'albendazole, le thiabendazole, le parbendazole, le cambendazole, l'oxibendazole, le mébendazole et le fluobendazole, et une quantité non toxique d'un sel de sélénium choisi parmi le sélénate de sodium, le sélénate de potassium, le sélénate d'ammonium, le sélénite de sodium, le sélénite de potassium, le sélénite d'ammonium, le séléniure de sodium, le séléniure de potassium et le séléniure d'ammonium, efficace pour prévenir les carences en sélénium chez le mouton et la chèvre, dans laquelle ledit agent anthelmintique est présent en une quantité allant de 100 à 400 parties en poids; et dans laquelle ledit sel de sélénium est présent en une quantité de 1 à 20 parties en poids, calculée en sélénium élémentaire, la préparation comprenant en outre de préférence une quantité non toxique d'un sel de cobalt efficace pour prévenir une carence en cobalt chez le mouton et la chèvre, ledit sel de cobalt étant présent en une quantité de 1 à 50 parties en poids, calculée en cobalt élémentaire.

7. Préparation vétérinaire pour bovins, comprenant une quantité efficace d'un agent anthelmintique choisi parmi le fenbendazole, l'oxfendazole, l'albendazole, le thiabendazole, le parbendazole, le cambendazole, l'oxibendazole, le mébendazole et le flubendazole, et une quantité non-toxique d'un sel de sélénium choisi parmi le sélénate de sodium le sélénate de potassium, le sélénate d'ammonium, le sélénite de sodium le sélénite de potassium, le sélénite d'ammonium, le séléniure de sodium, le séléniure de potassium et le séléniure d'ammonium, efficace pour prévenir une carence en sélénium chez un bovin, dans laquelle ledit agent anthelmintique est présent en une quantité allant de 150 à 1500 parties en poids, et dans laquelle ledit sel de sélénium est présent suivant une quantité de 0,75 à 18,75 parties en poids calculée en sélénium élémentaire, la préparation comprenant en outre de préférence une quantité non toxique d'un sel de cobalt efficace pour prévenir une carence en cobalt chez un bovin, ledit sel de cobalt étant présent suivant une quantité de 0,75 à 37,5 parties en poids, calculée en cobalt élémentaire.

8. Nécessaire vétérinaire, comprenant un agent anthelmintique suivant la revendication 1 et un sel de sélénium suivant la revendication 1, ledit agent anthelmintique et ledit sel de sélénium étant conditionnés séparément, et ledit agent anthelmintique et ledit sélénium étant adaptés pour être combinés afin de former une préparation vétérinaire comprenant une quantité efficace de l'agent anthelmintique et une quantité non toxique du sel de sélénium, efficace pour prévenir une carence en sélénium chez un animal, le nécessaire comprenant en outre de préférence un sel de cobalt, ledit sel de cobalt étant conditionné séparément du sel de sélénium et dudit agent anthelmintique et ledit agent anthelmintique, le sel de cobalt et le sel de sélénium étant adaptés pour être combinés afin de former une préparation vétérinaire comprenant une quantité efficace d'un agent anthelmintique, une quantité non toxique d'un sel de sélénium efficace pour prévenir une carence en sélénium chez un animal et une quantité non toxique d'un sel de cobalt efficace pour prévenir une carence en cobalt chez un animal.

9. Nécessaire vétéinaire pour les moutons et les chèvres, comprenant un agent anthelmintique suivant la revendication 1 et un sel de sélénium suivant la revendication 1, ledit agent anthelmintique et ledit sel de sélénium étant conditionnés séparément, et ledit agent anthelmintique et ledit sel de sélénium étant adaptés pour être combinés afin de former une préparation vétérinaire pour moutons et chèvres, comprenant une quantité efficace de l'agent anthelmintique et une quantité non toxique du sel de sélénium, efficace pour prévenir une carence en sélénium; nécessaire dans lequel ledit agent anthelmintique est présent en une quantité de 100 à 400 parties en poids et ledit sel de sélénium en une quantité de 1 à 20 parties en poids, calculée en sélénium élémentaire, le nécessaire comprenant en outre de préférence un sel de cobalt, ledit sel de cobalt étant conditionné séparément dudit sel de sélénium et dudit agent anthelmintique, ledit agent anthelmintique, le sel de cobalt et le sel de sélénium étant adaptés pour être combinés afin de former une préparation vétérinaire pour moutons et chèvres, comprenant une quantité efficace d'un agent anthelmintique, une quantité non toxique d'un sel de sélénium efficace pour prévenir une carence en sélénium chez un animal et une quantité non toxique d'un sel de cobalt efficace pour prévenir une carence en cobalt chez un animal, ledit sel de cobalt étant présent dans ledit nécessaire en une quantité de 1 à 50 parties en poids, calculée en cobalt élémentaire.

10. Nécessaire vétérinaire pour bovins comprenant un agent anthelmintique suivant la revendication 1, et un sel de sélénium suivant la revendication 1, ledit agent anthelmintique et ledit sel de sélénium étant condtionnés séparément, et ledit agent anthelmintique et ledit sel de sélénium étant adaptés pour être combinés afin de former une préparation vétérinaire pour bovins comprenant une quantité efficace de l'agent anthelmintique et une quantité non toxique du sel de sélénium, efficace pour prévenir une carence en sélénium chez un animal; nécessaire dans lequel ledit agent anthelmintique est présent en une quantité de 150 à 1500 parties en poids et ledit sel de sélénium en une quantité de 0,75 à 18,75 parties en poids, calculée en sélénium élémentaire, le nécessaire comprenant en outre de préférence un sel de cobalt, ledit sel de cobalt étant conditionné séparément dudit sel de sélénium et dudit agent anthelmintique, et ledit agent anthelmintique, le sel de cobalt et le sel de sélénium étant adaptés pour être combinés afin de former une préparation vétérinaire pour bovins comprenant une quantité efficace d'un agent anthelmintique, une quantité non toxique d'un sel de sélénium efficace pour prévenir une carence en sélénium chez un animal

18

et une quantité non toxique d'un sel de cobalt efficace pour prévenir une carence en cobalt chez un animal, ledit sel de cobalt étant présent dans ledit nécessaire suivant une quantité de 0,75 à 37,5 parties en poids, calculée en cobalt élémentaire.